Europäisches Patentamt

European Patent Office

Office européen des brevets

(19)

(11) Publication number: **0 078 331**
**B1**

(12) # EUROPEAN PATENT SPECIFICATION

(45) Date of publication of patent specification: **29.01.86**

(51) Int. Cl.⁴: **A 61 K 39/395**

(21) Application number: **81109312.9**

(22) Date of filing: **29.10.81**

(54) **Process for preparing immunoglobulin suitable for intravenous injection.**

(43) Date of publication of application:
**11.05.83 Bulletin 83/19**

(45) Publication of the grant of the patent:
**29.01.86 Bulletin 86/05**

(84) Designated Contracting States:
**AT BE CH DE FR GB LI LU NL SE**

(56) References cited:
**DE-A-2 364 792**
**GB-A-1 435 816**
**US-A-3 597 409**
**US-A-3 763 135**
**US-A-3 808 189**
**US-A-4 000 121**
**US-A-4 124 576**
**US-A-4 164 495**
**US-A-4 165 370**

(73) Proprietor: **THE GREEN CROSS CORPORATION**
**15-1, Imabashi-1-chome Higashi-ku Osaka-shi**
**Osaka 541 (JP)**

(72) Inventor: **Uemura, Yahiro**
**5-18, Mitsuyacho**
**Hirakata-shi (JP)**
Inventor: **Goto, Takashi**
**24-9 Kitanakaburi-1-chome**
**Hirakata-shi (JP)**
Inventor: **Kano, Yoshiaki**
**7-7, Sekime-2-chome**
**Joto-ku Osaka (JP)**
Inventor: **Funakoshi, Satoshi**
**16-5, Aoyama-1-chome**
**Katano-shi (JP)**

(74) Representative: **Vossius Vossius Tauchner**
**Heunemann Rauh**
**Siebertstrasse 4 P.O. Box 86 07 67**
**D-8000 München 86 (DE)**

Courier Press, Leamington Spa, England.

## Description

This invention relates to a process for preparing immunoglobulin suitable for intravenous injection.

Since immunoglobulins possess antibody activity to many pathogens, they are administered to patients deficient in various antibodies to protect them from infectious diseases or for therapeutic purposes. Two types of immunoglobulin preparations have been developed, one being for intravenous injection and the other for intramuscular injection, and both are widely used clinically.

The known methods for the preparation of immunoglobulin suitable for intravenous administration include those in which crude immunoglobulin is treated with proteases such as pepsin and plasmin, those in which chemical modification of immunoglobulin by acylation and other chemical means is employed, and those which fractionation of the plasma or of Cohn's plasma fractions using polyethylene glycol, Pluronics® (polyoxyethylene-polyoxypropylene copolymer, Wyandotte Chemicals Co.) are used.

In the immunoglobulin for intravenous administration obtained by pepsin treatment, 7S IgG is transformed into F(ab')₂ by the digestive action of pepsin, but the resulting compound has the disadvantage of a very short half-life in vivo. In the plasmin treatment, although 60 to 70% of the immunoglobulin remain in the form of normal immunoglobulin, the remainder is converted into low molecular substances which decrease the half-life in vivo of the product as in the case of pepsin treatment. In the case of chemical modification, there is the possibility of the development of fresh antigenicity depending upon the type of modifying radical and other conditions, bringing about the problem of safety when the modified immunoglobulin is repeatedly administered.

The fractionation with polyethylene glycol or Pluronics® is generally believed to be the most desirable means for recovering the immunoglobulin in the form existing in the living body, that is, unmodified and undecomposed immunoglobulin. The procedure has already been disclosed in detail by Polson and Coval [Japanese Patent Application "Kokai" (Laid-open) Nos. 46,814/1975, 91,321/1976 and 20,415/1978]. However, if the polyethylene glycol or Pluronic® fractionation is carried out under known conditions, although it is possible to obtain an immunoglobulin preparation containing no aggregate-type immunoglobulin and suitable for intravenous injection, the yield is always low. Therefore, an improvement in the yield has been eagerly awaited.

The present inventors conducted studies to elucidate the cause of the decrease in yield and found that in the step of removing the aggregate-type immunoglobulin at a low concentration of polyethylene glycol or a Pluronic® added to the raw immunoglobulin material, the non-aggregate-type immunoglobulin is also removed at the same time. Further, it also found that when an immunoglobulin material containing a comparatively large amount of aggregate-type immunoglobulin is fractionated with polyethylene glycol or a Pluronic®, the loss in non-aggregate-type immunoglobulin is also great. The present inventors further found that the yield of non-aggregate-type immunoglobulin is remarkably improved by treating the raw immunoglobulin material with an acid to dissociate the aggregate-type immunoglobulin and then subjecting the material to the fractionation with polyethylene glycol or a polyoxyethylene-polyoxypropylene copolymer. Based on this finding, the present invention has been accomplished.

An object of this invention is to provide a process for preparing in a high yield an immunoglobulin suitable for use in intravenous injection from a plasma or Cohn's fraction I+II+III, fraction II+III, fraction II, or fraction III obtained by subjecting a plasma to Cohn's cold alcohol fractionation.

Other objects and advantages of this invention will become apparent from the following description.

According to this invention there is provided a process for preparing an immunoglobulin suitable for intravenous injection, which comprises (a) adding at pH 4.6 to 5.4 to plasma or the Cohn's fraction I+II+III, fraction II+III, fraction II, or fraction III obtained by subjecting a plasma to Cohn's cold alcohol fractionation an alkylene oxide polymer or copolymer having a molecular weight of 2,000 to 20,000 to a concentration of 4.5 to 5.5% (W/V); (b) removing an aggregate-type immunoglobulin as a precipitate; (c) adding to the supernatant of step (b) said polymer or copolymer at pH 8.0 to 9.0 to a concentration of 6 to 13% (W/V) to recover as a precipitate a non-aggregate-type immunoglobulin containing substantially no aggregate-type immunoglobulin, characterized in that before step (a) the plasma or Cohn fractions are treated at 4° to 15°C for 30 to 180 minutes with an acid at pH 3.2 to 5.0 at an ionic strength of 0.002 to 0.30.

The plasma used as starting material according to this invention is preferably that originating from human blood in view of the problem of antigenicity. The Cohn's plasma fractions I+II+III, II+III, II and III are substantially α-, β- and γ-globulin (IgG, IgA, IgM). The plasma γ-globulin fraction is obtained by continuous precipitation with cold ethanol as described in detail in Journal of Clinical Investigation, *23*, 417 (1944) and Journal of the American Chemical Society, *68*, 459 (1946).

The acid treatment is carried out by keeping the starting material under acidic conditions. By this treatment, the aggregate-type immunoglobulin in the raw material is dissociated into a non-aggregate type. The acid conditions are pH 3.2 to 5.0, preferably 3.8 to 4.2 and an ionic strength of 0.002 to 0.30, preferably 0.10 to 0.20. Other pH conditions are undesirable because if the pH is below 3.2, denaturation of the protein will take place, while if it is above 5.0, the said dissociation of the aggregate-type immunoglobulin is insufficient. The concentration of protein is not critical, but is preferably 2 to 10% (W/V), because of the ease of operation. The temperature of the acid treatment is 4° to 15°C. Other temperatures are not desirable, because if the temperature is below 4°C, the aforesaid dissociation will be insufficient, while if it exceeds 15°C, decomposition of the protein will take place. The duration of the acid treatment is 30 to 180 minutes. If the duration of treatment is shorter than 30 minutes, the aforesaid dissociation is insufficient, while too long a duration of treatment is a waste of time and sometimes even gives unfavorable results. The acids used for the treatment include inorganic acids such as hydrochloric acid and phosphoric acid and organic acids such as acetic acid and citric acid.

After the acid treatment as described above, a fractionation is performed using an alkylene oxide polymer or copolymer having a molecular weight of 2,000 to 20,000, to produce a high-purity non-aggregate-type immunoglobulin in a high yield. The alkylene group of the alkylene oxide polymer used in the fractionation is one having 1 to 4 carbon atoms such as methylene, ethylene, propylene or butylene group. The alkylene oxide copolymers include copolymers of two or more alkylene oxides such as polyoxyethylene-polyoxypropylene copolymer.

The present inventors found that in the fractionation using an alkylene oxide polymer or copolymer having a molecular weight of 2,000 to 20,000, the pH is a very important factor and the yield and purity of the immunoglobulin is markedly improved only in the very limited pH range of from 4.6 to 5.4, preferably from 4.8 to 5.2. This is clearly shown also by the experimental results (Table 1) obtained by dissolving a fraction II+III paste in 0.6% aqueous sodium chloride solution to a protein concentration of 5%, treating the resulting solution with an acid at pH 3.5 at 10°C for 60 minutes, then fractionating the solution with polyethylene glycol (5% in concentration) at various pHs in the range of 4.0 to 6.0, and determining the yield and purity of the non-aggregate-type immunoglobulin.

TABLE 1
pH conditions in polyethylene glycol fractionation

| pH | IgG recovery, % | IgG purity, % |
|-----|-----------------|---------------|
| 4.1 | 60 | 35 |
| 4.3 | 58 | 60 |
| 5.0 | 70 | 98 |
| 5.5 | 45 | 95 |
| 6.0 | 10 | 93 |

When a polyoxyethylene-polyoxypropylene copolymer of an average molecular weight of 15,000 is used in place of the polyethylene glycol, higher yields and higher purity of the non-aggregate-type immunoglobulin are also obtained, under the above pH conditions than those obtained under other pH conditions.

The above polymer or copolymer is added to the starting material to a concentration of 4.5 to 5.5%, preferably 5% (W/V) and the precipitated impurities, e.g. aggregate-type immunoglobulin, are removed by customary means, e.g. centrifuging at 1,000—5,000 rpm (i.e. 5,000—10,000×g). To the thus fractionated supernatant the above polymer or copolymer is then added to a concentration of 6 to 13%. The intended non-aggregate-type immunoglobulin is then precipitated by adjusting the pH to 8.0 to 9.0, and recovered by customary means, e.g. centrifuging at 1,000 to 5,000 rpm (5,000—10,000×g). The recovery of the desired product under the above conditions is 60% or more. The obtained precipitate is again dissolved, for example, in a physiological saline or 0.02 M acetate buffer solution admixed with 0.6% of sodium chloride, 2% of mannit and 1% of albumin, and the resulting solution is passed through a bacterial filter to obtain an immunoglobulin solution suitable for intravenous administration. The immunoglobulin in this solution shows no change in its properties upon dispensing the solution in small portions into vials and lyophilizing. According, when the product is intended for long-term storage, it can be in the form of lyophilized preparation.

The immunoglobulin prepared by the present process contains substantially no aggregate-type immunoglobulin and the anticomplementary activity is less than 20 units, as assayed on a 5% solution the purity as IgG being 90% or above.

The invention is illustrated in detail with reference to the following non-limiting Examples.

In the Examples, the recovery of immunoglobulin was determined by single immuno-diffusion and the purity by electrophoresis using a cellulose acetate membrane. The anticomplementary activity was assayed by the method of Kabat and Mayer [Experimental Immunochemistry, p 225 (1961)] and the method of Nishioka and Okada ["Biochemistry of Immunity", p 103 (1971), Kyoritsu Publishing Company].

Example 1
The Cohn's fraction II+III paste (1 kg) obtained by cold alcohol fractionation was dissolved in 10 liters of a 0.6% aqueous sodium chloride solution. The solution was adjusted to pH 3.8 with 1N hydrochloric acid and stirred at 4°C for 60 minutes to effect acid treatment. To the solution was added 500 g of polyethylene glycol (average molecular weight 4,000). While allowing the polyethylene glycol to dissolve, pH of the solution was gradually increased with 1N aqueous sodium hydroxide solution. As soon as the pH reached 5.0, the precipitate was removed by centrifugation (4,000 rpm) to obtain a clear supernatant. The recovery of IgG in the supernatant was 85% based on the fraction II+III and the purity of IgG was 97%. To the supernatant was added another 500 g of polyethylene glycol (molecular weight 4,000). While being gently stirred, the pH of the supernatant was subjected to 8.5 with 1N aqueous sodium hydroxide solution. The immunoglobulin precipitated under the above conditions was recovered by centrifugation at 4,000 rpm (8000×g). The whole of the recovered precipitate was dissolved in a 0.02 M acetate buffer solution (pH 6.6) containing 1% of human albumin and using the same solvent the concentration was adjusted to 5%. The solution was sterilized by passing through a Millipore® filter (Millipore Co.) and aseptically dispensed into small containers. One half of the dispensed liquor was immediately lyophilized to yield a dry preparation.

The ultimate yield of IgG from the starting material was 84%, in comparison with 63% when the acid treatment at pH 3.8 was omitted. The purity was 95%, excluding the albumin which was added afterwards. The anticomplementary activities (at a protein concentration of 5%) of the liquid preparation and the solution of lyophilized preparation were found to be 14 and 16, respectively.

A 5% solution was administered to 5 mice, about 20 g in body weight, at a dose of 1 ml per mouse. Neither decrease in body weight nor any anomaly in piloerection was noted during an observation period of one week. The lyophilized preparation was tested after one year of storage at 4°C, but no change in solubility and anticomplementary activity was observed as compared with the initial preparation.

Example 2

A Cohn's fraction II paste (500 g) obtained by cold ethanol fractionation was dissolved in 10 liters of a 0.1% sodium chloride solution. Immunoglobulin containing no aggregate type was recovered from the solution in a manner similar to that in Example 1. The yield of IgG was 80%, in comparison with 62% when the acid treatment (pH 3.8) was omitted. The purity was 97%.

In a manner similar to that in Example 1, the immunoglobulin obtained above was dissolved in a 0.5% sodium chloride solution to a protein concentration of 5%. After addition of 1% of mannit, the solution was passed through a bacterial filter and lyophilized. The lyophilized preparation was dissolved in distilled water for injection to a protein concentration of 5%. The anticomplementary activity at this concentration was found to be 13.

Example 3

The procedure of Example 1 was repeated, except that the polyethylene glycol (average molecular weight 4,000) was replaced by the same amount of a polyoxyethylene-polyoxypropylene copolymer (average molecular weight 15,000). The immunoglobulin was recovered with the same results as in Example 1.

Claims

1. A process for preparing an immunoglobulin suitable for intravenous injection, which comprises:

(a) adding at pH 4.6 to 5.4 to plasma or the Cohn's fraction I+II+III, fraction II+III fraction II or fraction III obtained by subjecting a plasma to Cohn's cold alcohol fractionation an alkylene oxide polymer or copolymer having a molecular weight of 2,000 to 20,000 to a concentration of 4.5 to 5.5% (W/V);

(b) removing the aggregate-type immunoglobulin as a precipitate;

(c) adding to the supernatant of step (b) said polymer or copolymer at pH 8.0 to 9.0 to a concentration of 6 to 13% (W/V) to recover as a precipitate a non-aggregate-type immunoglobulin containing substantially no aggregate-type immunoglobulin, characterized in that before step (a) the plasma or Cohn's fractions are treated at 4° to 15°C for 30 to 180 minutes with an acid at pH 3.2 to 5.0 at an ionic strength of 0.002 to 0.30.

2. A process according to Claim 1, wherein the protein concentration in the acid treatment is 2 to 10% (W/V).

3. A process according to Claim 1, wherein the acid is hydrochloric acid, phosphoric acid, acetic acid, or citric acid.

4. A process according to Claim 1, wherein the plasma is one originating from human blood.

5. A process according to Claim 1, wherein the alkylene oxide polymer is one having methylene, ethylene, propylene or butylene groups.

6. A process according to Claim 1, wherein the alkylene oxide copolymer is a polyoxyethylene-polyoxypropylene copolymer.

Patentansprüche

1. Verfahren zur Herstellung eines für die intravenöse Injektion anwendbaren Immunglobulins, wobei man

(a) bei einem pH-Wert von 4,6 bis 5,4 Plasma oder die Fraktion I+II+III, Fraktion II+III, Fraktion II oder Fraktion III, die durch Plasmafraktionierung nach Cohn mit kaltem Alkohol erhalten worden ist, mit einem Alkylenoxid-Polymer oder -Copolymerisat mit einem Molekulargewicht von 2000 bis 20 000 bis zu einer Konzentration von 4,5 bis 5,5% (Gew./Vol.) versetzt,

(b) Immunglobulin-Aggregate als Fällung abtrennt,

(c) den Überstand der Stufe (b) mit dem Polymer oder Copolymerisat bei einem pH-Wert von 8,0 bis 9,0 bis zu einer Konzentration von 6 bis 13% (Gew./Vol.) versetzt und eine Fällung von aggregatfreiem Immunglobulin wiedergewinnt, die praktisch keine Immunglobulin-Aggregate enthält, dadurch gekennzeichnet, daß man vor der Stufe (a) das Plasma oder die Cohn-Fraktionen bei 4 bis 15°C während 30 bis 180 Minuten mit einer Säure bei einem pH-Wert von 3,2 bis 5,0 bei einer Ionenstärke von 0,002 bis 0,30 behandelt.

2. Verfahren nach Anspruch 1, dadurch gekennzeichnet, daß die Protein-Konzentration bei der Säurebehandlung 2 bis 10% (Gew./Vol.) beträgt.

3. Verfahren nach Anspruch 1, dadurch gekennzeichnet, daß die Säure Salzsäure, Phosphorsäure, Essigsäure oder Citronensäure ist.

4. Verfahren nach Anspruch 1, dadurch gekennzeichnet, daß das Plasma aus menschlichem Blut stammt.

5. Verfahren nach Anspruch 1, dadurch gekennzeichnet, daß das Alkylenoxid-Polymer Methylen-Äthylen-, Propylen- oder Butylengruppen enthält.

6. Verfahren nach Anspruch 1, dadurch gekennzeichnet, daß das Alkylenoxid-Copolymerisat ein Polyoxyäthylen-Polyoxypropylen-Copolymerisat ist.

## Revendications

1. Procédé de préparation d'immunoglobuline pour l'injection intraveineuse qui comprend:

(a) l'addition, à un pH de 4,6 à 5,4, au plasma ou aux fractions de Cohn I+II+III, aux fractions II+III à la fraction II ou à la fraction III, obtenues en soumettant un plasma au fractionnement par l'alcool froid de Cohn, d'un polymère ou d'un copolymère d'oxyde d'alkylène ayant un poids moléculaire de 2 000 à 20 000, à une concentration allant de 4,5 à 5,5% (P/V);

(b) la séparation de l'immunoglobuline de type agrégat sous forme d'un précipité;

(c) l'addition au surnageant du stade (b) dudit polymère ou copolymère à une concentration de 6 à 13% (P/V), à pH 8,0 à 9,0 pour recueillir sous forme d'un précipité une immunoglobuline de type non-agrégat ne contenant pas d'immunoglobuline de type agrégat, caractérisé par le fait que, avant le stade (a), le plasma ou les fractions de Cohn sont traités pendant 30 à 180 minutes, à une température allant de 4° à 15°C, par une acide, à un pH de 3,2 à 5,0 et à une force ionique de 0,002 à 0,30.

2. Procédé selon la revendication 1, dans lequel la concentration de protéine dans le traitement acide est de 2 à 10% (P/V).

3. Procédé selon la revendication 1, dans lequel l'acide est l'acide chlorhydrique, l'acide phosphorique, l'acide acétique ou l'acide citrique.

4. Procédé selon la revendication 1, dans lequel le plasma est un plasma provenant de sang humain.

5. Procédé selon la revendication 1, dans lequel le polymère d'oxyde d'alkylène est un polymère d'oxyde d'alkylène contenant des groupes méthylène, éthylène, propylène ou butylène.

6. Procédé selon la revendication 1, dans lequel le copolymère d'oxyde d'alkylène est un copolymère polyoxyéthylène-polyoxypropylène.